# EUROPEAN PATENT SPECIFICATION

(11) **EP 2 623 052 B1**
(45) Date of publication and mention of the grant of the patent: **22.01.2020**
(21) Application number: 11828029.6
(22) Date of filing: 24.08.2011
(51) Int. Cl.: A61B 17/3209

(54) **SURGICAL MILLING CUTTER**
CHIRURGISCHES FRÄS- UND SCHNEIDEWERKZEUG
FRAISE CHIRURGICALE

(30) Priority: 30.09.2010 CN 201010298082
(43) Date of publication of application: 07.08.2013
(73) Proprietor: Chongqing Ruzer Pharmaceutical Co., Ltd., Chongqing 401120 (CN)
(72) Inventor: YE, Lei, Chongqing 401120 (CN); ZHOU, Jian, Chongqing 401120 (CN); FENG, Hua, Chongqing 401120 (CN); LI, Fei, Chongqing 401120 (CN); ZHU, Hengyang, Chongqing 401120 (CN); LI, Congxiao, Chongqing 401120 (CN)
(74) Representative: Stöckeler, Ferdinand
(86) International application number: PCT/CN2011/078817
(87) International publication number: WO 2012/041133

(56) References cited:
- EP-A2- 0 317 503
- CN-A- 101 926 672
- CN-U- 201 814 633
- CN-Y- 201 211 208
- CN-Y- 201 211 208
- CN-Y- 201 414 828
- DE-U1- 20 304 155
- US-A- 4 936 313
- US-A1- 2007 123 891

## Description

### Field of the Invention

The invention relates to a surgical cutting instrument and, more particularly, to a surgical milling cutter.

### Description of the Prior art

During an operation, a milling cutter is used to perform some work of cutting. The milling cutter includes a milling cutter bracket, a locking apparatus, and an electric motor in top-down sequence. An L-shaped bracket having a hole provided on the short side is provided on the top of the milling cutter bracket, a cylindrical bit in the front of the milling cutter bit projecting into the hole, which ensures a preferred force bearing effect with the milling cutter. To protect the milling cutter, the cylindrical bit partially projects into the hole, leaving a part outside the hole. In prior art, the short side of the L-shaped bracket is designed to be a plane structure. When cutting an object, the cylindrical bit would not function if it contacts the object, thus causing uneven cutting to the object. Additionally, the structure makes it difficult for the milling cutter to turn direction during the cutting process, which brings inconvenience to using the milling cutter.

Document CN201211208 Y discloses a surgical milling cutter which forms the basis of the preamble of the claim 1.

### Summary of the Invention

Claim 1 defines the invention and the dependent claims disclose the preferred embodiments. The object of the invention is to provide a surgical milling cutter, which has the cylindrical bit not contacting the object to be cut and which can turn direction flexibly.

To achieve the object, the following technical scheme is adopted.

A surgical milling cutter includes a milling cutter bracket, a locking apparatus and a bottom electric motor connected sequentially, the surgical milling cutter bracket includes a retaining base with a through hole, characterized in that a finger guide with an L-shaped first bracket at its top is disposed at the upper part of the retaining base, a downward protrusion is disposed at the end of the short side of the L-shaped first bracket, and the lowest point of the protrusion is lower than the lowest point of a cylindrical bit of the milling cutter during normal operation of the milling cutter;
a bolt with a through hole in its center is fixed in the cavity of the finger guide; a pressing plate is disposed at the upper part of the retaining base, and the nut passes through the hole of the pressing plate to connect to the bolt.

The locking apparatus includes: a flange with a through hole, a bearing is disposed at the inner wall of a small cylinder above the flange, a locking sleeve is provided over the flange, a protrusion is disposed on the inner wall of the upper part of the locking sleeve, and a conical surface is formed at the inner wall at the lower part of the locking sleeve; a spring is disposed between the bottom surface of the conical surface and the upper bottom surface of the flange, and a pressing plate is threadedly connected to the outer wall on the top of the small cylinder above the flange and presses the protrusion; a conical profile corresponding to the conical surface is made on the wall of the small cylinder above the flange, and a steel ball is disposed in the conical profile.

To allow the transmission rod to bear uniform stress, two bearings are disposed below the outer wall and above the upper bottom surface.

To ensure the two bearings to be securely fixed, a supporting member is disposed between two bearings.

To facilitate the connection of the reducer, the inner wall of the big cylinder below the flange is provided with the inner threads,

A bolt with a through hole at its center is fixed in the cavity of the finger guide; a pressing plate is disposed at the upper part of the retaining base, and a nut passes through the hole of the pressing plate to connect to the bolt.

A finger guide is disposed at the lower part of the retaining base, a T-shaped bolt is fixed in the cavity of the finger guide, a pressing plate is disposed at the upper part of the retaining base, and the nut passes through the hole of the pressing plate to connect to the bolt.

To ensure the smooth motion of the milling cutter, a bearing is disposed at the upper part of the finger guide.

To avoid the excessively flexibility of the finger guide, a spring sleeves the bolt.

To prevent skidding, the outer surfaces of the retaining base and finger guide are both provided with an anti-slip groove.

The invention adopts the protrusion to prevent the object from contacting the cylindrical bit, thus to avoid cutting the object unevenly. Meanwhile, the finger guide may drive the L-shaped bracket to rotate to change the motion direction of the milling cutter, which facilitates the operation.

### Brief Description of the Drawings

FIG.1 is a structural diagram showing the whole structure in an embodiment of the invention.
FIG.2 is a structural diagram of the milling cutter bracket in FIG.1.
FIG.3 is a structural diagram of the locking apparatus in FIG.1.

### Detailed Description of the Preferred Embodiments

The invention is described in detail with the appended drawings and the embodiment.

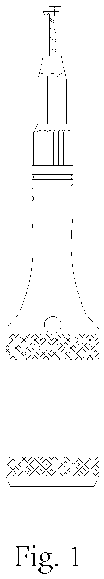

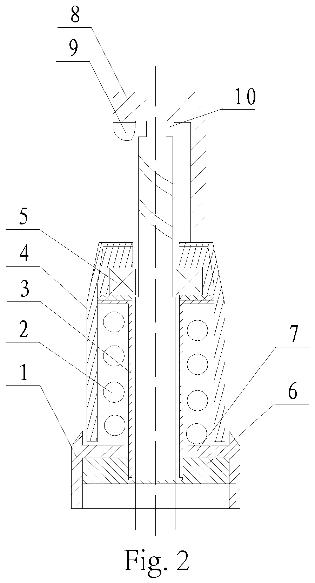

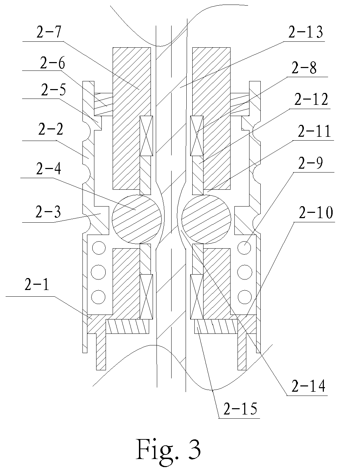

As shown in FIG.1 to FIG.3, a surgical milling cutter includes a retaining base 1 with a through hole. A finger guide 4 with an L-shaped first bracket at its top is disposed at the upper part of the retaining base 1. A protrusion 9 is extending downwardly from the end of the short side 8 of the L-shaped first bracket, and the lowest point of the protrusion 9 is lower than the lowest point of the cylindrical bit 10 during normal operation of the milling cutter. Thus, it is impossible for the object to contact the cylindrical bit, thus to avoid uneven cutting of the object. A T-shaped bolt 3 with a through hole is fixed in the cavity of the finger guide 4. A pressing plate 7 is disposed at the upper part of the retaining base 1, and the nut 6 passes through the hole of the pressing plate 7 to connect to the bolt 3.

The locking apparatus includes a flange 2-1 with a through hole, and a bearing 2-8 is disposed on the inner wall of the small cylinder above the flange. Two bearings 2-8 are disposed below the outer wall 2-7 and above the upper bottom surface 2-10, respectively. A supporting member 2-12 is disposed between the two bearings 2-8. The two bearings may effectively ensure the smooth motion of the transmission rod to reduce the generated heat. A pressing member 2-15 is threadedly connected to the upper bottom surface 2-10 to press against the bearing 2-8 and the supporting member 2-12 to secure the bearing 2-8 and prevent it from falling out. A locking sleeve 2-2 is sleeved onto the flange. A protrusion 2-5 is disposed on the inner wall at the upper part of the locking sleeve 2-2, and a conical surface 2-3 is formed at the inner wall at the lower part of the locking sleeve 2-2. A spring 2-9 is disposed between the bottom surface of the conical surface 2-3 and the upper bottom surface 2-10 of the flange. A pressing plate 2-6 is threadedly connected to the outer wall 2-7 on the top of the small cylinder above the flange and presses the protrusion 2-5. The conical profile 2-11 communicating with the through hole of the flange is made on the wall of the small cylinder above the flange. The steel ball 2-4 is disposed in the conical profile 2-11, and the groove 2-14 corresponding to the conical profile 2-11 is formed on the transmission rod 2-13 of the milling cutter. The inner wall of the big cylinder below the flange is provided with the inner threads. In this embodiment, the number of the conical holes 2-11 and the steel balls 2-4 are two. As an equivalent modification, the number also may be three, thus to obtain a better locking effect. By adopting this locking structure, when the transmission rod needs to be inserted, the locking sleeve just needs to the pressed. Then the conical surface moves down, and the steel balls roll outwards. After the transmission rod reaches the designated position, the locking sleeve is released. Under the effect of the spring, the locking sleeve is raised, and conical surface presses against the steel ball to move inward to snap into the snap-slot on the transmission rod which is thus secured. The locking or releasing process is easy and convenient in this locking status, providing a good fixing effect and an improved safety is offered.

A bearing 5 is disposed at the upper part of the finger guide 4. A spring 2 is sleeved on the bolt 3. The outer surfaces of the retaining base 1 and finger guide 4 are both provided with anti-slip grooves. The bearing 5 provides the milling cutter with a near force point to ensure the smooth motion of the milling cutter. The spring 2 ensures that a certain force exists between the finger guide 4 and the retaining base, thus to avoid misoperation caused by the excessively flexible rotation of the finger guide 4.

When the finger guide 4 is rotated, the bolt 3 and the nut 6 are rotated therewith. Thus, the finger guide 4 drives the L-shaped bracket to rotate to change the motion direction of the milling cutter to facilitate the operation.

The above-mentioned is merely embodiment of the invention and not to be construed in a limiting sense. Without departing from the scope of the invention, various changes, modifications or equal replacements in equivalent embodiments according to the above disclosure will become apparent to those skilled in this art. All simple changes, equal replacements and modifications which fall within the scope of the invention as defined by the claims are seen to fall within the scope of the invention.

## Claims

1. A surgical milling cutter, comprising:
a milling cutter bracket,
a cutter locking apparatus, and
a proximal electric motor which are connected sequentially,
wherein the milling cutter bracket comprises a retaining base (1) with a through hole,
wherein a distal L-shaped bracket (8) is provided, the free end of the short section having a protrusion (9), the protrusion having a most proximal point that is more proximal than the most proximal point of a distal cylindrical section (10) of the milling cutter during normal operation of the milling cutter, and
wherein the cutter locking apparatus comprises a flange (2-1) with a through hole, a bearing (2-8) is disposed at the inner wall of a small cylinder (2-7) distal to the flange, a locking sleeve (2-2) is provided over the flange, a protrusion (2-5) is disposed on the inner wall at the distal part of the locking sleeve (2-2), and a conical surface (2-3) is formed at the inner wall at the proximal part of the locking sleeve (2-2); a spring (2-9) is disposed between the proximal surface of the conical surface (2-3) and the distal surface of the proximal part (2-10) of the flange, and a pressing plate (2-6) is threadedly connected to the outer wall (2-7) on the distal section of the small cylinder (2-7) distal to the flange and abbuts the protrusion (2-5) disposed on the inner wall of the distal part of the locking sleeve; a conical profile (2-11) corresponding to the conical surface (2-3) is provided on the wall of the small cylinder distal to the flange, and a steel ball (2-4) is disposed in the conical profile (2-11)
**characterized in that**
a rotatable finger guide (4) is attached to the distal part of the retaining base (1), and wherein the L-shaped bracket is attached to the distal end of the finger guide (4), such that the protrusion (9)extends proximally from the free end of the short side (8) of the L-shaped bracket;
a bolt (3) with a center through hole is fixed in a cavity of the finger guide (4);
a pressing plate (7) is disposed at the distal end of the retaining base (1), and a nut (6) is located proximal to a hole in the pressing plate (7) for connecting to the bolt (3).

2. The surgical milling cutter according to claim 1, **characterized in that** the number of the bearings (2-8) is two, and they are disposed within the outer wall of the small cylinder (2-7) distal to the flange and distal to the proximal surface (2-10) of the flange, respectively.

3. The surgical milling cutter according to claim 2, **characterized in that** a supporting member (2-12) is disposed between the two bearings (2-8).

4. The surgical milling cutter according to any of claims 1 to 3, **characterized in that** the inner wall of a cylinder projecting from the proximal surface of the flange (2-1) is provided with inner threads.

5. The surgical milling cutter according to claim 4, **characterized in that** a bearing (5) is disposed at the distal part of the finger guide (4).

6. The surgical milling cutter according to claim 5, **characterized in that** a spring (2) surrounds the bolt (3).

7. The surgical milling cutter according to claim 6, **characterized in that** the outer surfaces of the retaining base (1) and finger guide (4) are both provided with at least one anti-slip groove.

## Patentansprüche

1. Ein chirurgischer Fräser, der folgende Merkmale aufweist.
eine Fräserhalterung,
eine Fräserverriegelungsvorrichtung, und
einen proximalen Elektromotor, die der Reihe nach verbunden sind,
wobei die Fräserhalterung eine Zurückhaltungsbasis (1) mit einem Durchgangsloch aufweist,
wobei eine distale L-förmige Halterung (8) bereitgestellt ist, wobei das freie Ende des kurzen Abschnitts einen Vorsprung (9) aufweist, der Vorsprung einen proximalen Punkt aufweist, der während eines Normalbetriebs des Fräsers proximaler ist als der proximalste Punkt eines distalen zylindrischen Abschnitts (10) des Fräsers, und
wobei die Fräserverriegelungsvorrichtung einen Flansch (2-1) mit einem Durchgangsloch aufweist, ein Lager (2-8) an der Innenwand eines kleinen Zylinders (2-7) distal zu dem Flansch angeordnet ist, eine Verriegelungshülse (2-2) über dem Flansch bereitgestellt ist, ein Vorsprung (2-5) auf der Innenwand an dem distalen Teil der Verriegelungshülse (2-2) angeordnet ist, und eine konische Oberfläche (2-3) an der Innenwand an dem proximalen Teil der Verriegelungshülse (2-2) gebildet ist; wobei eine Feder (2-9) zwischen der proximalen Oberfläche der konischen Oberfläche (2-3) und der distalen Oberfläche des proximalen Teils (2-10) des Flansches angeordnet ist, und eine Druckplatte (2-6) gewindemäßig mit der Außenwand (2-7) an dem distalen Bereich des kleinen Zylinders (2-7) distal zu dem Flansch verbunden ist und gegen den Vorsprung (2-5), der auf der Innenwand des distalen Teils der Verriegelungshülse angeordnet ist, anstößt; wobei ein konisches Profil (2-11), das der konischen Oberfläche (1-3) entspricht, auf der Wand des kleinen Zylinders distal zu dem Flansch bereitgestellt ist, und eine Stahlkugel (2-4) in dem konischen Profil (2-11) angeordnet ist,
**dadurch gekennzeichnet, dass**
eine drehbare Fingerführung (4) an dem distalen Teil der Zurückhaltungsbasis (1) angebracht ist,
und wobei die L-förmige Halterung an dem distalen Ende der Fingerführung (4) derart angebracht ist, dass sich der Vorsprung (9) proximal von dem freien Ende der kurzen Seite (8) der L-förmigen Halterung erstreckt;
ein Bolzen (3) mit einem mittleren Durchgangsloch in einem Hohlraum der Fingerführung (4) befestigt ist;
eine Druckplatte (7) an dem distalen Ende der Zurückhaltungsbasis (1) angeordnet ist und sich eine Mutter (6) proximal zu einem Loch in der Druckplatte (7) befindet, um mit dem Bolzen (3) verbunden zu werden.

2. Der chirurgische Fräser gemäß Anspruch 1, der **dadurch gekennzeichnet ist, dass** die Anzahl der Lager (2-8) zwei beträgt und dieselben in der Außenwand des kleinen Zylinders (2-7) distal zu dem Flansch beziehungsweise distal zu der proximalen Oberfläche (2-10) des Flansches angeordnet sind.

3. Der chirurgische Fräser gemäß Anspruch 2, der **dadurch gekennzeichnet ist, dass** ein Stützbauglied (2-12) zwischen den zwei Lagern (2-8) angeordnet ist.

4. Der chirurgische Fräser gemäß einem der Ansprüche 1 bis 3, der **dadurch gekennzeichnet ist, dass** die Innenwand eines Zylinders, der von der proximalen Oberfläche des Flansches (2-1) hervorsteht, mit einem inneren Gewinde versehen ist.

5. Der chirurgische Fräser gemäß Anspruch 4, der **dadurch gekennzeichnet ist, dass** ein Lager (5) an dem distalen Teil der Fingerführung (4) angeordnet ist.

6. Der chirurgische Fräser gemäß Anspruch 5, der **dadurch gekennzeichnet ist, dass** eine Feder (2) den Bolzen (3) umgibt.

7. Der chirurgische Fräser gemäß Anspruch 6, der **dadurch gekennzeichnet ist, dass** die Außenoberflächen der Zurückhaltungsbasis (1) und der Fingerführung (4) beide mit zumindest einer Antirutschrille versehen sind.

## Revendications

1. Fraise chirurgicale, comprenant:
un support de fraise,
un appareil de verrouillage de fraise, et
un moteur électrique proximal qui sont connectés en séquence,
dans laquelle le support de fraise comprend une base de retenue (1) avec un trou traversant,
dans laquelle est prévu un support distal en "L" (8), l'extrémité libre du segment court présentant une saillie (9), la saillie présentant un point le plus proximal qui est plus proximal que le point le plus proximal d'un segment cylindrique distal (10) de la fraise pendant le fonctionnement normal de la fraise, et
dans laquelle l'appareil de verrouillage de fraise comprend une bride (2-1) avec un trou traversant, un palier (2-8) est disposé sur la paroi intérieure d'un petit cylindre (2-7) distal par rapport à la bride, un manchon de verrouillage (2-2) est prévu sur la bride, une saillie (2-5) est disposée sur la paroi intérieure dans la partie distale du manchon de verrouillage (2-2), et une surface conique (2-3) est formée sur la paroi intérieure dans la partie proximale du manchon de verrouillage (2-2); un ressort (2-9) est disposé entre la surface proximale de la surface conique (2-3) et la surface distale de la partie proximale (2-10) de la bride, et une plaque de pression (2-6) est connectée par un filetage à la paroi extérieure (2-7) sur le segment distal du petit cylindre (2-7) du côté distal par rapport à la bride et vient en butée contre la saillie (2-5) disposée sur la paroi intérieure de la partie distale du manchon de verrouillage; un profilé conique (2-11) correspondant à la surface conique (2-3) est prévu sur la paroi du petit cylindre du côté distal par rapport à la bride, et une bille en acier (2-4) est disposée dans le profilé conique (2-11),
**caractérisée par le fait que**
un guide-doigt rotatif (4) est fixé à la partie distale de la base de retenue (1),
et dans laquelle le support en "L" est fixé à l'extrémité distale du guide-doigt (4), de sorte que la saillie (9) s'étende du côté proximal par rapport à l'extrémité libre du petit côté (8) du support en "L";
un boulon (3) avec un trou traversant central est fixé dans une cavité du guide-doigt (4);
une plaque de pression (7) est disposée à l'extrémité distale de la base de retenue (1), et un écrou (6) est situé proximal par rapport à un trou dans la plaque de pression (7) pour la connexion au boulon (3).

2. Fraise chirurgicale selon la revendication 1, **caractérisée par le fait que** le nombre de roulements (2-8) est de deux, et qu'ils sont disposés à l'intérieur de la paroi extérieure du petit cylindre (2-7) respectivement du côté distal par rapport à la bride et du côté distal par rapport à la surface proximale (2-10) de la bride.

3. Fraise chirurgicale selon la revendication 2, **caractérisée par le fait qu'**un élément de support (2-12) est disposé entre les deux roulements (2-8).

4. Fraise chirurgicale selon l'une quelconque des revendications 1 à 3, **caractérisée par le fait que** la paroi intérieure d'un cylindre ressortant de la surface proximale de la bride (2-1) est munie de filets intérieurs.

5. Fraise chirurgicale selon la revendication 4, **caractérisée par le fait qu'**un palier (5) est disposé dans la partie distale du guide-doigt (4).

6. Fraise chirurgicale selon la revendication 5, **caractérisée par le fait qu'**un ressort (2) entoure le boulon (3).

7. Fraise chirurgicale selon la revendication 6, **caractérisée par le fait que** les surfaces extérieures de la base de retenue (1) et du guide-doigt (4) sont toutes deux munies d'au moins une rainure antidérapante.
